# EUROPÄISCHE PATENTSCHRIFT

(11) **EP 2 185 680 B1**
(45) Veröffentlichungstag und Bekanntmachung des Hinweises auf die Patenterteilung: **29.05.2013**
(21) Anmeldenummer: 08802304.9
(22) Anmeldetag: 17.09.2008
(51) Int. Cl.: C12M 1/34, C12M 1/42, C12M 3/00

(54) **VORRICHTUNG UND VERFAHREN ZUR KONDITIONIERUNG BIOLOGISCHER ZELLEN**
DEVICE AND METHOD FOR CONDITIONING BIOLOGICAL CELLS
DISPOSITIF ET PROCÉDÉ DE CONDITIONNEMENT DE CELLULES BIOLOGIQUES

(30) Priorität: 28.09.2007 DE 102007046516
(43) Veröffentlichungstag der Anmeldung: 19.05.2010
(73) Patentinhaber: Fraunhofer-Gesellschaft zur Förderung der angewandten Forschung e.V., 80686 München (DE)
(72) Erfinder: FUHR, Günter, 13187 Berlin (DE); ZIMMERMANN, Heiko, 66386 St.Ingbert (DE)
(74) Vertreter: Hertz, Oliver
(86) Internationale Anmeldenummer: PCT/EP2008/007780
(87) Internationale Veröffentlichungsnummer: WO 2009/043484

(56) Entgegenhaltungen:
- WO-A-2005/083057
- WO-A-2006/059109
- US-B1- 6 541 243
- US-B1- 6 991 906

## Beschreibung

Die Erfindung betrifft eine Konditionierungsvorrichtung für biologische Zellen mit den Merkmalen des Oberbegriffs von Anspruch 1, insbesondere eine Konditionierungsvorrichtung zur Konditionierung, z. B. Manipulation, Stimulation, Prägung und/oder Differenzierung, biologischer Zellen durch eine Wechselwirkung mit einer Konditionierungsprobe. Hauptanwendungen der Konditionierungsvorrichtung bestehen bei der Konditionierung von biologischen Zellen und der Untersuchung von deren Reaktionen nach einer Berührung durch die Konditionierungsprobe. Des Weiteren betrifft die Erfindung Konditionierungsverfahren, die insbesondere mit der Konditionierungsvorrichtung realisierbar sind.

Biologische Zellen, insbesondere tierische oder menschliche Zellen, stehen im Organismus permanent mit benachbarten Zellen, biologischen Oberflächen oder Flüssigkeiten in Wechselwirkung. Dabei finden zwischenmolekulare Wechselwirkungen zwischen Oberflächenmolekülen benachbarter Zellen oder zwischen Oberflächenmolekülen einer Zelle mit benachbarten Substanzen statt. Es können molekulare Oberflächenbindungen ausgetauscht und in den beteiligten Zellen molekulare Signalketten induziert werden. Die Signalketten setzen sich z. B. in das Zytoplasma fort, wo sie Vorgänge der Zellentwicklung, wie z. B. die Genexpression beeinflussen (siehe J. Gerhart in "Theratology", Bd. 60, 1999, S. 226-239).

Durch Oberflächen-Wechselwirkungen biologischer Zellen kann zum Beispiel eine Zelldifferenzierung ausgelöst oder beeinflusst werden kann. Des Weiteren kann sich die Oberflächen-Wechselwirkung auf die Bildung von Gewebe aus biologischen Zellen auswirken. In ausgeprägter Form sind Prozesse des Signalaustausches und einer sog. Zellsynchronisierung insbesondere bei der Zelldifferenzierung oder der Wundheilung zu beobachten. Speziell die Differenzierung und Vermehrung von Stammzellen beruht auf der Bindung von Oberflächenrezeptoren der Stammzellen an Liganden. Während der Embryogenese oder einer Regeneration von Gewebe (z. B. Wundheilung) werden Veränderungen von biologischen Zellen durch Oberflächenkontakte induziert.

Biologisch wirksame Moleküle, d. h. Moleküle, die bei Kontakt mit einer biologische Zelle, insbesondere der Zelloberfläche, eine Veränderung der biologischen Zelle induzieren, werden auch als Signalfaktoren bezeichnet. Es werden lösliche Signalfaktoren, die z. B. in einem Kultivierungsmedium oder einer Körperflüssigkeit enthalten sind, und oberflächengebundene Signalfaktoren unterschieden, die z. B. auf Zelloberflächen oder auf Oberflächen von Aggregaten biologischer Stoffe oder Grenzflächen im biologischen Organismus angeordnet sind.

Die in vitro-Untersuchung der Veränderung biologischer Zellen nach einer Berührung mit einem Signalfaktor stellt aus den folgenden Gründen ein extrem komplexes Problem dar. Erstens kann jede Berührung einer Zelle mit einem Molekül in der Umgebung eine biologisch wirksame Wechselwirkung induzieren. Jede Berührung kann für die Zelle eine molekulare Information darstellen, so dass Signalketten mit Änderungen von Zellzuständen ausgelöst werden. Für eine Untersuchung der physiologisch relevanten Signalfaktoren müssen durch die Schaffung definierter Umgebungsbedingungen der Zelle molekulare Fehlinformationen vermieden werden, wie sie z. B. durch eine Berührung mit einer künstlichen Oberfläche, wie Glas, Metall oder Kunststoff auftreten kann. Derartige künstliche Oberflächen sind jedoch bei den herkömmlichen Techniken der Zellbiologie, z. B. als Teile von Kulturgefäßen oder Instrumenten, permanent präsent. Zweitens wird die in vitro-Untersuchung komplex, da eine große Anzahl von Signalfaktoren und Signalketten bekannt sind, die sich spezifisch auf Zellen auswirken. Um die Wirkung von einem bestimmten Signalfaktor oder von Kombinationen aus Signalfaktoren zu testen, sind extrem viele Parametervariationen erforderlich, bspw. um einen der heute bekannten 17 Signalwege der höheren tierischen Zellen zu identifizieren, für die jeweils einige 10 bis zu einige 100 Signalfaktoren wirksam sind (siehe obige Publikation von J. Gerhart).

Ein bekanntes Konzept für die Konditionierung und *in vitro-*Untersuchungen biologischer Zellen basiert darauf, die Zellen während eines Tests ausschließlich in einer physiologischen Lösung oder auf einer Oberfläche anzuordnen, die von der Zelle als physiologische Oberfläche oder zumindest nicht als künstliche Oberfläche erkannt wird. Dabei erfolgt eine berührungslose Manipulation der Zellen, z. B. durch dielektrophoretische oder optische Kräfte, um unerwünschte Oberflächenkontakte zu vermeiden.

Ein gattungsgemäßes Konditionierungsverfahren zur Umsetzung dieses Konzepts ist bspw. aus US 6 991 906 B1 bekannt. Bei dieser Technik, die schematisch in Figur 9 illustriert ist, wird eine erste Zelle 1 mit einer Halteeinrichtung 10' umfassend eine Gruppe von Elektroden zur Bildung eines dielektrophoretischen Feldkäfigs 12' gehaltert. Eine optische Pinzette 20' wird zur Halterung einer zweiten Zelle 2 und zu deren Bewegung hin zur ersten Zelle 1 verwendet. Die zweite Zelle 2 bildet eine Probe zur Stimulation der ersten Zelle 1. Durch die Bewegung des Fokus der optischen Pinzette 20' kann die zweite Zelle 2 mit der ersten Zelle 1 in Kontakt gebracht werden. Nach einer Wechselwirkung kann eine Untersuchung der ersten Zelle 1 oder eine Trennung der Zellen 1, 2 unter Verwendung der optischen Pinzette 20' erfolgen.

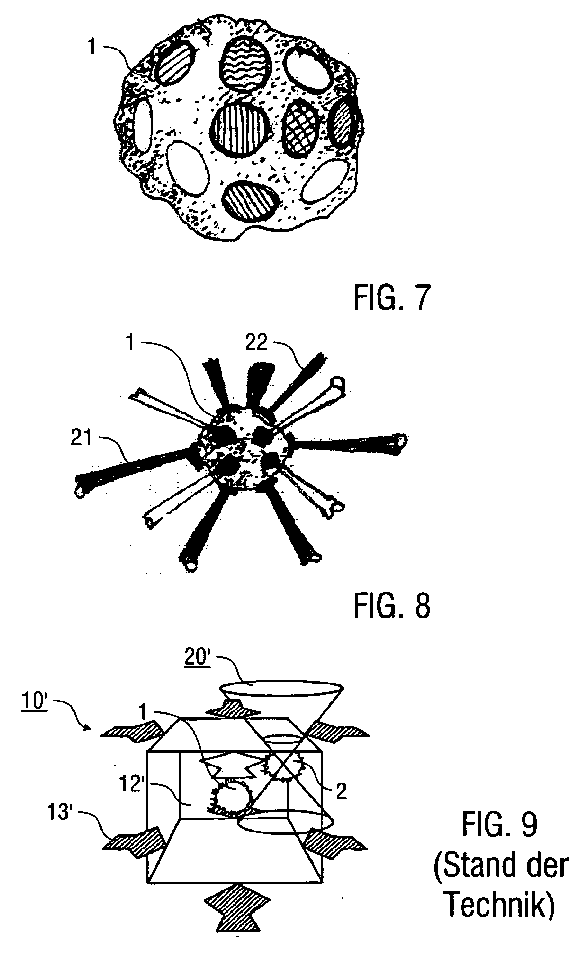

Die Technik gemäß Figur 9 hat eine Reihe von Nachteilen, durch die eine praktische Anwendung für in vitro-Untersuchungen beschränkt wird. Ein erster Nachteil ergibt sich daraus, dass nur einzelne Zell-Zell-Wechselwirkungen untersucht werden können. Für eine Annäherung von zwei Zellen (oder anderen Proben) an die Zelle 1 wären zwei optische Pinzetten erforderlich, die sich jedoch gegenseitig stören würden. Des Weiteren kann die Zelle 2 nur aus bestimmten Richtungen, insbesondere von oben zu der Zelle 1 bewegt werden, da andernfalls die optische Pinzette 20' durch die Elektroden 13' oder die Zelle 1 abgeschattet wird. Nachteilig kann auch sein, dass eine hohe Lichtintensität im Fokus der optischen Pinzette 20' einen unerwünschten Einfluss auf die Zellen oder deren Wechselwirkungen hat. Schließlich besteht ein Nachteil in den geringen Fangkräften der optischen Pinzette 20'. Zwei Zellen 1, 2 können nach der gegenseitigen Berührung und Bildung zwischenmolekularer Wechselwirkungen mit der optischen Pinzette 20' nicht mehr problemlos voneinander getrennt werden.

Eine weitere gattungsgemäße Technik zur Umsetzung des oben genannten Konzepts ist in WO 2005/083057 beschrieben. Bei dieser Technik wird die zu untersuchende Zelle auf einem magnetischen Träger angeordnet, der unter der Wirkung eines Magnetfeldes auf dem Boden eines mit Flüssigkeit gefüllten Kanals verschiebbar ist. Eine weitere Zelle, die mit der ersten Zelle in Wechselwirkung gebracht werden soll, kann auf einem weiteren magnetischen Träger angeordnet sein, der insbesondere an einem Deckelement des Kanals gegenüber zu dem Boden verschiebbar angeordnet ist. Durch eine passende Ausrichtung der magnetischen Träger können die Zellen miteinander zur Wechselwirkung gebracht werden. Auch die in WO 1005/083057 beschriebene Technik hat den Nachteil, dass nur einzelne Zell-Zell-Wechselwirkungen untersucht werden können. Die geometrische Ausdehnung der magnetischen Träger verhindert, dass mehrere Zellen voneinander unabhängig mit einer zu untersuchenden Zelle in Kontakt gebracht werden. Ein weiterer Nachteil besteht darin, dass nur einseitige Zellberührungen möglich sind. Die für die Untersuchung zur Verfügung stehende Zelloberfläche ist durch die Ablage auf dem magnetischen Träger stark beschränkt. Schließlich besteht ein Nachteil darin, dass nur globale Zell-Zell-Wechselwirkungen untersucht werden können. Der Zellkontakt kann bei Verwendung der magnetischen Träger nicht auf bestimmte Teile der Zelloberfläche beschränkt werden.

Somit sind die bisherigen Techniken zur Umsetzung des oben genannten Konzepts für in vitro-Untersuchungen auf spezielle Anwendungen beschränkt (z. B. Untersuchungen auf planaren Oberflächen, einseitige Zellbehandlung). Ein Werkzeug für die lösungs- und oberflächenbezogene Handhabung von biologischen Zellen, das genaue und zuverlässige Tests auch für komplexe Kombinationen von Signalfaktoren ermöglicht, ist jedoch bisher nicht verfügbar.

Die Aufgabe der Erfindung ist es, eine verbesserte Konditionierungsvorrichtung zur Handhabung biologischer Zellen, insbesondere für Test-, Manipulations- oder Untersuchungszwecke bereitzustellen, mit der die Nachteile und Beschränkungen der herkömmlichen Techniken vermieden werden. Die Aufgabe der Erfindung ist es ferner, ein verbessertes Konditionierungsverfahren bereitzustellen, das für eine *in vitro*-Konditionierung biologischer Zellen geeignet ist.

Diese Aufgaben werden durch eine Konditionierungsvorrichtung und ein Konditionierungsverfahren mit den Merkmalen der nebengeordneten Ansprüche gelöst. Vorteilhafte Ausführungsformen und Anwendungen der Erfindung ergeben sich aus den abhängigen Ansprüchen.

Gemäß einem ersten Gesichtspunkt beruht die Erfindung auf der allgemeinen technischen Lehre, eine gattungsgemäße Konditionierungsvorrichtung mit einer Halteeinrichtung zur Halterung von mindestens einer biologischen Zelle und einer Kontakteinrichtung zur Halterung und Bewegung mindestens einer Konditionierungsprobe derart, dass diese die mindestens eine biologische Zelle berührt, so weiterzubilden, dass die Kontakteinrichtung mindestens zwei mechanische Trägerelemente aufweist, die relativ zur Halteeinrichtung beweglich sind. Die Trägerelemente tragen die mindestens eine Konditionierungsprobe auf ihren freien Enden, die zum Haltebereich weisen. Die Trägerelemente sind vorzugsweise aus verschiedenen Raumrichtungen, insbesondere unter verschiedenen Raumwinkeln von mehreren Seiten hin zu der Halteeinrichtung und von dieser weg beweglich.

Durch die Bereitstellung der mindestens zwei Trägerelemente wird die Funktionalität der Konditionierungsvorrichtung im Vergleich zur herkömmlichen Technik vorteilhafterweise erheblich erweitert. So können die Trägerelemente der Kontakteinrichtung in Abhängigkeit von der konkreten Testaufgabe verschiedene Funktionen erfüllen. Ein oder mehrere Trägerelemente können eine oder verschiedene Konditionierungsproben tragen und mit diesen zu einem Haltebereich der Halteeinrichtung geschoben werden, während ein weiteres Trägerelement an den Haltebereich angrenzend angeordnet ist, um einen mechanischen Anschlag für die mindestens eine Zelle im Haltebereich während der Berührung durch die anderen Trägerelemente zu bilden. Gemäß einer anderen Variante können alle Trägerelemente eine oder verschiedene Konditionierungsproben tragen, die mit der mindestens einen Zelle im Haltebereich in Kontakt gebracht werden.

Mit dem Begriff "Konditionierung" wird hier die Bereitstellung einer vorbestimmten molekularen Wechselwirkung einer Zelle mit einer Probe (Konditionierungsprobe) bezeichnet. Die Konditionierung kann allgemein auch als Stimulation, Prägung oder Differenzierung bezeichnet werden. Mit der Konditionierung erfolgt eine gezielte Nachbildung eines realen Vorgangs (molekulare Wechselwirkung der Zelle mit der Umgebung) in einer Modellumgebung, die in der Konditionierungsvorrichtung gebildet ist.

Ein weiterer wichtiger Vorteil der Erfindung ergibt sich daraus, dass die Trägerelemente mechanische Komponenten darstellen, die aus einem Festkörpermaterial gebildet sind. Die mindestens eine Konditionierungsprobe wird abweichend von der herkömmlichen Technik (z. B. gemäß Figur 9) nicht berührungslos, sondern mit mechanisch übertragenen Antriebskräften bewegt. Vorteilhafterweise kann damit die Genauigkeit der Positionierung der Konditionierungsprobe bei der Berührung der Zelle im Haltebereich verbessert werden. Des Weiteren wird eine gegenseitige Wechselwirkung der Trägerelemente, wie sie z. B. bei optischen Pinzetten auftreten würde, ausgeschlossen. Schließlich können mit den erfindungsgemäß verwendeten Trägerelementen im Vergleich zur herkömmlichen Technik vergrößerte Kräfte bei der Berührung der Konditionierungsprobe mit der Zelle oder bei deren Trennung ausgeübt werden.

Ein weiterer wichtiger Vorteil der Konditionierungsvorrichtung besteht darin, dass die Trägerelemente entlang verschiedener Raumrichtungen beweglich sind. Die Bewegung entlang verschiedener Raumrichtungen bedeutet, dass die Bewegungsrichtungen von jeweils zwei der mindestens zwei mechanischen Trägerelemente eine Ebene aufspannen, die auch den Haltebereich der Halteeinrichtung enthält. Es ist vorteilhafterweise eine mehrseitige Anordnung der Trägerelemente an der Zelle im Haltebereich vorgesehen, wobei freie Enden der Trägerelemente (sog. Stempelflächen) zum Haltebereich weisen. Vorteilhafterweise wird durch die mehrseitige Anordnung (Anordnung auf mehreren Seiten der Zelle) eine gegenseitige Störung der Bewegung der Trägerelemente und/oder eine gegenseitige Beeinflussung von ggf. verschiedenen Konditionierungsproben auf den Trägerelementen ausgeschlossen. Die lang gestreckte, vorzugsweise gerade Form der Trägerelemente, die insbesondere an ihren freien, zum Haltebereich weisenden Ende eine Verjüngung (z.B. Konusform) aufweisen können, hat den zusätzlichen Vorteil, dass die Positionierung der Trägerelemente auf bestimmte, gewünschte Raumbereiche beschränkt wird.

Ein weiterer wichtiger Vorteil der Erfindung besteht darin, das in frei wählbarer Kombination parallel oder aufeinander folgend komplexe Oberflächenkontakte an Zellen realisiert werden können. Die Oberflächenkontakte können so gebildet sein, dass sie auf nur einen Teilbereich der Oberfläche der Zelle einwirken, wobei der Teilbereich in Abhängigkeit von der konkreten Anwendung der Erfindung wählbar ist. Des weiteren kann sichergestellt werden, dass zusätzlich zu den beabsichtigten Oberflächenkontakten keine unerwünschten, unspezifischen Oberflächenkontakte an der zu untersuchenden Zelle erfolgen.

Gemäß einem zweiten Gesichtspunkt beruht die Erfindung darauf, ein Konditionierungsverfahren zur Konditionierung mindestens einer biologischen Zelle mit mindestens einer Konditionierungsprobe insbesondere für eine anschließende Untersuchung der Zelle bereitzustellen, wobei bei dem Verfahren vorzugsweise die erfindungsgemäße Konditionierungsvorrichtung verwendet wird. Das Konditionierungsverfahren umfasst die Schritte der Bereitstellung der mindestens einen Zelle im Haltebereich der Halteeinrichtung und der mindestens einen Konditionierungsprobe auf mindestens einem der Trägerelemente sowie die Bewegung von mindestens einem der mindestens zwei Trägerelemente derart, dass die Zelle von mindestens einer Konditionierungsprobe berührt wird. Vorteilhafterweise kann die Zelle einer zeitlich und/oder örtlich determinierten Berührung durch die mindestens eine Konditionierungsprobe unterzogen werden. Die Konditionierung erfolgt vorzugsweise zerstörungsfrei, d. h. mit der mindestens einen Konditionierungsprobe können Signale, z. B. für die Zelldifferenzierung auf die Zelle ausgeübt werden, ohne die Zelle zu zerstören, insbesondere ohne die Zellmembran zu penetrieren.

Ein weiterer Vorteil der Erfindung besteht in der Variabilität bei der Gestaltung der Halteeinrichtung. So kann die Halteeinrichtung gemäß einer ersten Variante zur Halterung der mindestens einen biologischen Zelle mit berührungslos wirkenden Kräften, z. B. unter der Wirkung dielektrischer Kräfte oder optischer Kräfte eingerichtet sein. In diesem Fall, in dem die Halterung ohne Berührung fester Oberflächen erfolgen kann, umfasst die Halteeinrichtung bspw. einen dielektrischen Feldkäfig, wie er an sich aus der fluidischen Mikrosystemtechnik bekannt ist, oder eine optische Pinzette. Der Haltebereich wird entsprechend durch ein Feldminimum im Feldkäfig oder den Fokus der optischen Pinzette gebildet. Diese Variante der Erfindung hat den Vorteil, dass die Zelle mit an sich bekannten Methoden schonend in die Halteeinrichtung bewegt und dort positioniert werden kann. Das zum Beispiel bei der herkömmlichen Technik gemäß Figur 9 auftretende Problem verminderter Haltekräfte wird vermieden, da die Trägerelemente zur Ausübung von Halte-, Kontakt- und/oder Trennungskräften bei der Wechselwirkung der Zelle mit der Konditionierungsprobe verwendet werden.

Mit der berührungslosen Halterung der mindestens einen biologischen Zelle kann vorteilhafterweise ein Verfahren realisiert werden, bei dem zunächst die Zelle mit relativ schwachen Kräften eingefangen und gehalten wird. Anschließend erfolgt die Konditionierung unter Verwendung der mechanischen Trägerelemente, die danach unter Ausübung relativ starker Kräfte von der Zelle getrennt werden können.

Gemäß einer zweiten Variante der Erfindung kann die Halteeinrichtung mindestens eine Haltefläche aufweisen, auf der die mindestens eine biologische Zelle positionierbar ist. In diesem Fall erfolgt die Halterung nicht berührungslos, sondern in Kontakt mit der Haltefläche. Die Haltefläche kann z. B. Teil mindestens eines mechanischen Halteelements sein, das wie die Trägerelemente aufgebaut und beweglich ist. Des Weiteren kann die Haltefläche allgemein eine feste Oberfläche oder eine Grenzfläche zwischen zwei Flüssigkeiten verschiedener Viskosität, z. B. zwischen zwei Kultivierungsphasen sein. Die Positionierung der Zelle auf der Haltefläche kann in Bezug auf eine erhöhte Stabilität der ortsfesten Halterung in der Halteeinrichtung von Vorteil sein.

Die Haltefläche trägt vorzugsweise eine biokompatible Beschichtung, die für die Zelle in der Halteeinrichtung biochemisch unwirksam ist. Die biokompatible Beschichtung ist biochemisch unwirksam, wenn bei Ablage einer Zelle auf der Halteoberfläche und einer ggf. auftretenden zwischenmolekularen Wechselwirkung der Zelle mit der biokompatiblen Beschichtung keine Veränderung der Zelle oder eine vorbestimmte, bei der aktuellen Testaufgabe unkritische Veränderung induziert werden. Beispiele für biokompatible Beschichtungen, die für verschiedene Testaufgaben und verschiedene Zelltypen angewendet werden können, umfassen Fibronektin, Kollagen oder immobilisierte Moleküle von Signalfaktoren zur Differenzierung von Zellen, wie zum Beispiel IGF 1 (Insulin-like-Growth Factor), BMP2 (Bone morphogenetic protein 2), BMP7 (Bone morphogenetic protein 7), VEGF (Vascular Endothelial Growth Factor) oder TGFB3 (transforming growth factor).

Die Halteeinrichtung kann auch beide genannten Varianten verkörpern, also für eine Halterung der Zelle sowohl mit berührungslos wirkenden Kräften als auch mit einem mechanischen Kontakt mit der Haltefläche eingerichtet sein. Es kann z. B. vorgesehen sein, dass in einer ersten Phase (Bereitstellung der Zelle im Haltebereich) eine berührungslose Halterung und in einer zweiten Phase (Berührung der Zelle im Haltebereich mit der Konditionierungsprobe) eine Kontakthalterung mit der Haltefläche erfolgt.

Gemäß einer bevorzugten Ausführungsform der Erfindung ist die Halteeinrichtung in einem Behälter angeordnet, der zur Aufnahme eines flüssigen Kultivierungsmediums eingerichtet ist. Vorteilhafterweise können im Behälter mit dem Kultivierungsmedium vorbestimmte Kultivierungsbedingungen eingestellt werden. Dabei kann das Kultivierungsmedium zur Ernährung der mindestens einen Zelle in der Halteeinrichtung und/oder als zusätzliche Testsubstanz verwendet werden. Das Kultivierungsmedium kann z. B. einen (oder mehrere) Differenzierungsfaktor(en) enthalten, dessen (oder deren) Wirkung in Zusammenhang mit der Wirkung der mindestens einen Konditionierungsprobe auf den Trägerelementen untersucht wird.

Gemäß einer vorteilhaften Gestaltung der erfindungsgemäßen Konditionierungsvorrichtung weist der Behälter ein Innenvolumen auf, das mindestens gleich dem vierfachen Volumen der mindestens einen biologischen Zelle im Haltebereich ist. Typischerweise ist diese Bedingung erfüllt, wenn das Innenvolumen mindestens gleich dem vierfachen Volumen des Haltebereiches (z. B. Volumen des wirksamen Feldminimums im Feldkäfig) ist. Besonders bevorzugt ist das Innenvolumen größer als das 10- bis 100-fache Volumen der mindestens einen biologischen Zelle bzw. des Haltebereiches. Vorteilhafterweise kann damit ein ausreichend großer Kultivierungsraum für die mindestens eine biologische Zelle und auch Platz für Untersuchungen der Zelle, z. B. optische Beobachtungen oder Impedanzmessungen geschaffen werden. Besonders bevorzugt ist das Innenvolumen des Behälters im Bereich von 4 · 10³ µm³ bis 1000 mm³ gewählt.

Vorteilhafterweise kann der Behälter der erfindungsgemäßen Konditionierungsvorrichtung weitere Funktionen übernehmen, mit denen die Anwendbarkeit der Erfindung erheblich erweitert wird. So kann in einer Wand des Behälters mindestens ein Lager vorgesehen sein, dass als Führung für die Bewegung von mindestens einem der mechanischen Trägerelemente eingerichtet ist. Ein Lager für eines der mindestens zwei Trägerelemente kann zur Umsetzung der Erfindung ausreichend sein, wobei in diesem Fall das andere der Trägerelemente ohne ein Lager bewegt oder angeordnet wird. Bevorzugt ist jedoch eine Ausführung der Erfindung, bei der mehrere Lager in der Behälterwand vorgesehen sind, deren Anzahl gleich der Anzahl der mechanischen Trägerelemente ist. Vorteilhafterweise können in diesem Fall durch die Lager in der Behälterwand die Bewegungsrichtungen der Trägerelemente festgelegt werden. Des Weiteren kann mindestens ein Fluidkanal vorgesehen sein, der durch die Behälterwand führt und eine Fluidverbindung zwischen dem Innenraum des Behälters und einer Umgebung des Behälters, z. B. einem angeschlossenen Fluidiksystem herstellt. Der Fluidkanal kann vorteilhafterweise zum Transport der mindestens einen biologischen Zelle in die Halteeinrichtung verwendet werden. Des Weiteren kann der mindestens eine Fluidkanal für eine Zufuhr und/oder einen Austausch der Kultivierungsflüssigkeit im Behälter verwendet werden. Ferner kann der Behälter eine Messfunktion erfüllen, wenn er mit mindestens einem Sensor zur Erfassung einer physikalischen oder chemischen Größe im Behälter eingerichtet ist. Der Sensor kann z. B. einen Lichtsensor und/oder einen Impedanzsensor umfassen. Für Beleuchtungs- und Untersuchungszwecke kann es des Weiteren von Vorteil sein, wenn der Behälter mindestens ein Optikelement enthält, das in der Behälterwand vorgesehen ist, um Licht in das Innere des Behälters, insbesondere in den Haltebereich der Halteeinrichtung einzukoppeln und/oder Licht aus dem Haltebereich zu erfassen. Das Optikelement kann z. B. ein optisches Fenster in der Behälterwand, eine Abbildungsoptik und/oder Lichtleiter umfassen.

Gemäß einer weiteren bevorzugten Ausführungsform der Erfindung ist vorgesehen, dass mindestens eines der Trägerelemente, vorzugsweise jedoch alle Trägerelemente eine gerade Stabform aufweisen. Die Trägerelemente umfassen einen Trägerstab, der in den Lagern in der Behälterwand verschiebbar ist. Am freien Ende des Trägerstabs, das zum Haltebereich der Halteeinrichtung, d. h. zur biologischen Zelle weist, hat das Trägerelement eine Stempelfläche, die als Aufnahme der Konditionierungsprobe verwendet wird.

Vorzugsweise ist die Stempelfläche kleiner als die Oberfläche der mindestens einen biologischen Zelle, insbesondere kleiner als die Oberfläche des Haltebereichs. Eine bevorzugte Querschnittsfläche der Stempelfläche ist im Bereich von 1 µm² bis 10.000 µm² gewählt. Vorteilhafterweise ermöglicht dies in Abhängigkeit von der konkreten Untersuchungsaufgabe die gleichzeitige Berührung der Oberfläche der mindestens einen biologischen Zelle mit einer Vielzahl von Trägerelementen und/oder die Berührung bestimmter Oberflächenbereiche, z. B. in der Nähe von bestimmten Membranorganellen der Zelle.

Vorzugsweise weist die Stempelfläche eine konkave, konvexe oder ebene Form auf. Die konkave Form hat den Vorteil, das bei der Berührung der Stempelfläche mit der zu untersuchenden Zelle deren Membran aufgespannt wird, so dass ein verbesserter Oberflächenkontakt gebildet wird. Die konvexe Form kann Vorteile für einen Schutz der Konditionierungsprobe auf der Stempelfläche während der Bewegung des Trägerelements haben. Vorteile einer ebenen Form der Stempelfläche können sich insbesondere für bestimmte Zusatzfunktionen der Konditionierungsvorrichtung, z. B. bei der Formung einer Zellgruppe in der Halteeinrichtung ergeben. Alternativ oder zusätzlich kann die Stempelfläche eine Mikrostrukturierung aufweisen, wie z. B. Vorsprünge mit typischen Dimensionen im Bereich von 50 nm bis 5 µm. Gemäß einer weiteren Alternative kann auf der Stempelfläche eine Testbeschichtung vorgesehen sein, welche die Konditionierungsprobe des jeweiligen Trägerelements bildet. Die Testbeschichtung umfasst biologisch wirksame Moleküle, wie z. B. die oben genannten Signalfaktoren. In Abhängigkeit von der konkreten Testaufgabe können Kombinationen der genannten Formen, Strukturen und Testbeschichtungen vorgesehen sein.

Die Trägerelemente der Konditionierungsvorrichtung können bei Bedarf manuell bewegt werden. Gemäß einer weiteren vorteilhaften Ausführungsform ist die Konditionierungsvorrichtung jedoch mit einer Antriebseinrichtung ausgestattet, mit der mindestens eines der Trägerelemente beweglich ist. Die Antriebseinrichtung ist relativ zum Behälter der Konditionierungsvorrichtung ortsfest positioniert, so dass vorteilhafterweise eine genaue und reproduzierbare Bewegung der Trägerelemente realisiert werden kann. Besonders bevorzugt umfasst die Antriebseinrichtung mindestens einen piezoelektrischen Antrieb. Typischerweise ist eine Vielzahl piezoelektrischer Antriebe vorgesehen, deren Zahl der Anzahl der Trägerelemente und ggf. der Halteelemente entspricht. Jedes Trägerelement oder Halteelement ist mit einem eigenständigen piezoelektrischen Antrieb versehen, so dass vorteilhafterweise eine große Flexibilität bei der selektiven Betätigung einzelner Trägerelemente besteht.

Besonders bevorzugt ist die Antriebseinrichtung so gebildet, dass die Trägerelemente auf geraden Bewegungsbahnen verschiebbar sind, die in Bezug auf den Haltebereich radial ausgerichtet sind. Dies ermöglicht eine allseitige Kontaktierung der mindestens einen biologischen Zellen im Haltebereich. Durch eine Form der Trägerelemente, die sich zu ihren freien Enden hin konisch verjüngen, wird deren Bewegung auf geraden Bahnen bis zum Haltebereich unterstützt.

Die Umsetzung der Erfindung ist nicht auf die Bereitstellung von zwei Trägerelementen beschränkt. In Abhängigkeit von der konkreten Testaufgabe können mehr als zwei Trägerelemente, insbesondere mindestens 10 Trägerelemente, besonders bevorzugt mindestens 20 Trägerelemente, z. B. bis zu 50 oder 100 oder sogar mehr als 100 Trägerelemente vorgesehen sein. Durch die selektive Ansteuerung der einzelnen Trägerelemente kann eine hohe kombinatorische Vielfalt bei der Einstellung verschiedener Testbedingungen erzielt werden.

Zur Realisierung des erfindungsgemäßen Konditionierungsverfahrens kann es ausreichend sein, die zu untersuchende Zelle mit einer oder mehreren Konditionierungsproben zu kontaktieren. Nach der Konditionierung kann die Zelle aus der Konditionierungsvorrichtung entfernt und zunächst aufbewahrt und/oder einer Kultivierung unterzogen werden. Gemäß einer bevorzugten Ausführungsform der Erfindung ist jedoch vorgesehen, dass nach einer Kontaktierungsphase, bei der eine oder mehrere Konditionierungsproben gleichzeitig oder zeitlich versetzt mit der Zelle in Kontakt gebracht wurden, eine Untersuchung der Zelle vorgesehen ist. Vorteilhafterweise kann die Untersuchung in der Konditionierungsvorrichtung, z. B. durch optische oder elektrische Messungen nach an sich bekannten Messverfahren erfolgen. Alternativ kann die Zelle für die Untersuchung aus der Konditionierungsvorrichtung in ein getrenntes Messgerät überführt werden.

Gemäß einer weiteren Variante kann die konditionierte Zelle nach der Induktion einer Differenzierung einer weiteren Kultivierung (insbesondere Langzeitkultur) unterzogen werden, um Zellgruppen differenzierter Zellen zu bilden. Diese Zellgruppen können vorteilhafterweise bei Verfahren des "Tissue engineering" angewendet werden.

Ein weiterer Vorteil der Erfindung besteht in der hohen Variabilität bei der Auswahl der Konditionierungsprobe, die vorzugsweise in Abhängigkeit von der Testaufgabe Signalmoleküle (z. B. BMP2, BMP7, IGF), biologisch wirksame Makromoleküle (z. B. Vitamine, Hormone), biologische Zellen (z. B. differenzierte Zellen oder Vorläuferzellen), Zellbestandteile (z. B. Zellbestandteile von differenzierten Zellen, insbesondere Membranbestandteile) und/oder mikrostrukturierte Oberflächen umfasst.

Wenn gemäß einer weiteren Variante der Erfindung das mindestens eine Trägerelement mit einer Geschwindigkeit bewegt wird, die im Bereich von 5 µm/h bis 10 mm/h gewählt ist, so können sich Vorteile für eine physiologisch schonende Berührung der mindestens einen biologischen Zelle ergeben. Bei Geschwindigkeiten im genannten Intervall können die Zellen sich aufgrund ihrer natürlichen Reorganisation der molekularen Zellkontakte der Zellmembran an das sich annähernde Trägerelement anpassen. Vorteilhafterweise wird damit ein besonders effektiver Zellkontakt erzielt.

Vorteilhafterweise kann das erfindungsgemäße Konditionierungsverfahren mit weiteren Untersuchungs- und/oder Manipulationsschritten kombiniert werden. Die weiteren Schritte umfassen z. B. eine Bereitstellung einer Konditionierungsprobe als Komponente einer Kultivierungsflüssigkeit, welche die mindestens eine biologische Zelle umgibt, eine Bildung eines die mindestens eine biologische Zelle umgebenden Hohlraums, der durch Stempelflächen von Trägerelementen und/oder Halteflächen von Halteelementen eingeschlossen wird, eine Messung von Wechselwirkungskräften zwischen der Zelle und der Konditionierungsprobe, wobei molekulare Bindungskräfte der Zellmembran der untersuchten Zelle erfasst werden können, eine mechanische Deformation der mindestens einen biologischen Zelle, wobei elastische Eigenschaften des Zellplasmas erfasst und Rückschlüsse auf den physiologischen Zustand der Zelle ermöglicht werden, eine Flüssigkeitsbehandlung der mindestens einen biologischen Zelle im Haltebereich der Halteeinrichtung, eine Verkapselung der mindestens einen biologischen Zelle, und/oder eine Überführung der Zelle in einen gefrorenen Zustand.

Weitere Einzelheiten und Vorteile der Erfindung werden im Folgenden unter Bezug auf die beigefügten Zeichnungen beschrieben. Es zeigen:
- Figur 1:: eine erste Ausführungsform der erfindungsgemäßen Konditionierungsvorrichtung in schematischer Perspektivansicht;
- Figur 2:: eine Illustration einer Ausführungsform des erfindungsgemäßen Verfahrens, bei dem eine optische Untersuchung vorgesehen ist;
- Figur 3:: eine weitere Ausführungsform der erfindungsgemäßen Konditionierungsvorrichtung;
- Figuren 4 und 5:: weitere Einzelheiten von Trägerelementen einer erfindungsgemäßen Konditionierungsvorrichtung;
- Figur 6:: eine weitere Illustration einer Ausführungsform des erfindungsgemäßen Konditionierungsverfahrens;
- Figur 7:: eine schematische Illustration einer Zelloberfläche, die einem erfindungsgemäßen Test unterzogen wurde;
- Figur 8:: eine weitere Anwendung der erfindungsgemäßen Trägerelemente; und
- Figur 9:: eine Illustration eines herkömmlichen Konditionierungsverfahrens (Stand der Technik).

Die Erfindung wird im Folgenden insbesondere unter Bezug auf Einzelheiten der Halterung der zu untersuchenden Zelle und der Eigenschaften und Handhabung der Konditionierungsvorrichtung, insbesondere der Trägerelemente beschrieben. Einzelheiten der Kultivierung von Zellen oder Manipulation von Zellen unter der Wirkung hochfrequenter elektrischer Felder mittels negativer Dielektrophorese werden hier nicht beschrieben, da diese an sich aus dem Stand der Technik bekannt sind. Die Erfindung wird beispielhaft unter Bezug auf den Test mit einer einzigen Zelle erläutert. Die Umsetzung der Erfindung ist jedoch nicht auf diese Variante beschränkt. Ersatzweise können mehrere Zellen in der Halteeinrichtung angeordnet und als Zellaggregat einem Test wie eine einzelne Zelle unterzogen werden.

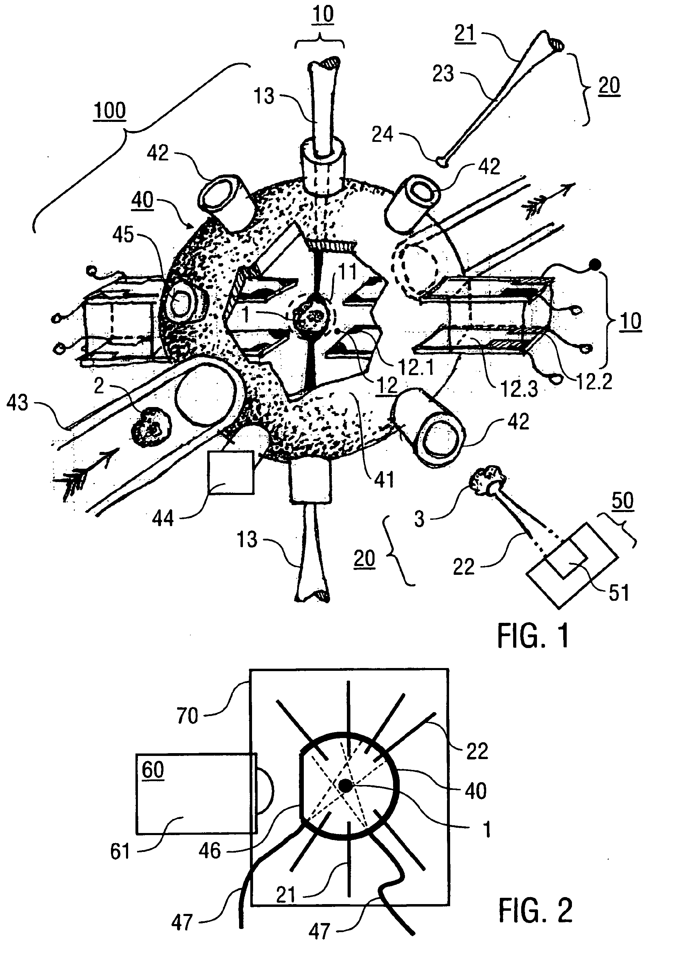

Figur 1 zeigt eine erste Ausführungsform einer erfindungsgemäßen Konditionierungsvorrichtung 100 mit einer Halteeinrichtung 10 und einer Kontakteinrichtung 20, die in oder an einem Behälter 40 mit einer Behälterwand 41 angebracht sind. Die Halteeinrichtung 10 weist einen Haltebereich 11 auf, der durch das Feldminimum eines dielektrischen Feldkäfigs 12 und/oder Halteelemente 13 gebildet wird.

Der dielektrische Feldkäfig 12 umfasst acht Elektroden 12.1, die über Elektrodenanschlüsse 12.2 mit einer Spannungsquelle (nicht dargestellt) verbunden sind. Die Elektroden 12.1 und die Elektrodenanschlüsse 12.2 sind auf Elektrodenträgern 12.3 angeordnet. Die Elektrodenträger 12.3 umfassen rechteckige Platten, die in schlitzförmigen Öffnungen des Behälters 40 verschiebbar angeordnet sind. Bei Beaufschlagung der Elektroden 12.1 mit hochfrequenten elektrischen Spannungen wird im dielektrischen Feldkäfig 12 das gewünschte Feldminimum gebildet, in dem sich eine Zelle 1 unter der Wirkung dielektrophoretischer Kräfte anordnet.

Die Halteelemente 13 der Halteeinrichtung 10 sind zusätzlich zur mechanischen Halterung der Zelle 1 vorgesehen. Die Halteelemente 13 umfassen bewegliche Stabelemente, die wie die Trägerelemente der Kontakteinrichtung 20 aufgebaut sind (siehe unten).

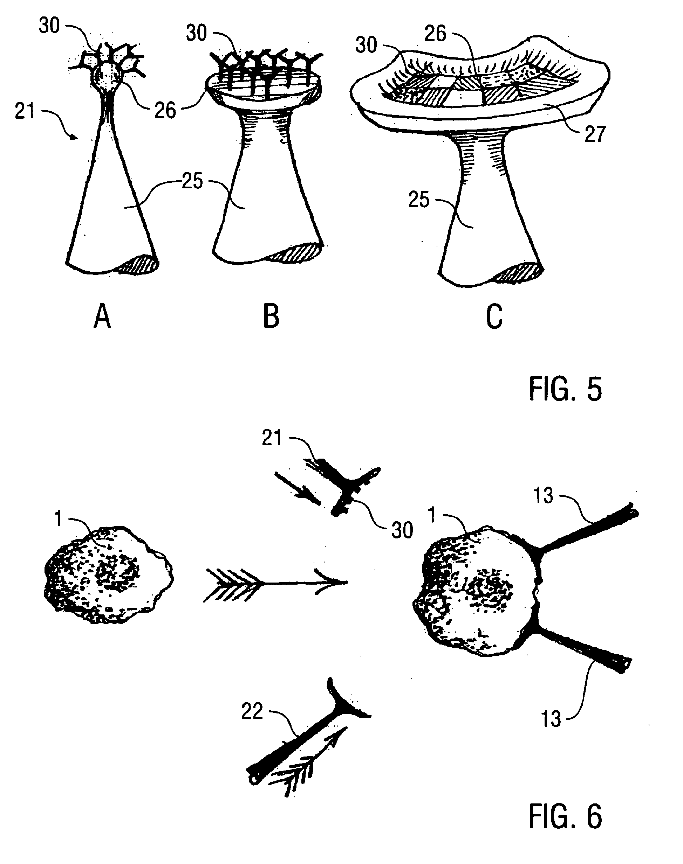

Die Kontakteinrichtung 20 umfasst eine Vielzahl von Trägerelementen, von denen in Figur 1 beispielhaft nur zwei Trägerelemente 21, 22 gezeigt sind. Jedes Trägerelement 21, 22, das einen Trägerstab 23 und eine Stempelfläche 24 (Einzelheiten siehe Figur 5) umfasst, ist in einem Lager 42 in der Behälterwand 41 verschiebbar angeordnet. Der Trägerstab 23 kann wie gezeigt eine konische, sich zur Stempelfläche 24 verjüngende Form aufweisen. Alternativ kann der Trägerstab 23 eine gerade, z. B. zylindrische Stabform aufweisen. Zur Bewegung der Trägerelemente 21, 22 ist eine Antriebseinrichtung 50 (beim Trägerelement 22 schematisch gezeigt) vorgesehen, die an jedem Trägerelement 21, 22 einen piezoelektrischen Antrieb 51 oder einen Stellmotor aufweist. Die Trägerelemente 21, 22 werden erfindungsgemäß in Richtung ihrer longitudinalen Ausdehnung (Stabform) bewegt.

Um ein Wandern von Zellen entlang der Trägerelemente aufgrund einer natürlichen Zellbewegung zu verhindern, werden die Trägerstäbe 25 vorzugsweise mit einer Beschichtung versehen, die eine Zelladhäsion behindert oder unterbindet. Die Beschichtung umfasst z. B. eine Hydrophobisierung, eine PTFE-Beschichtung oder eine durch eine Plasmabehandlung modifizierte Oberflächenschicht.

Die Behälterwand 41, die in Figur 1 zur Illustrationszwecken teilweise durchbrochen dargestellt ist, kann z. B. eine Kugelform bilden. Der Durchmesser des Behälters 40 kann in Abhängigkeit von der konkreten Anwendung der Erfindung im Bereich von z. B. 100 µm bis zu einigen Zentimetern gewählt sein. Je weniger Zellen getestet werden sollen, umso geringer wird das Kugelvolumen gewählt, damit die Bildung physiologischer Bedingungen im Inneren des Behälters 40 erleichtert und insbesondere ermöglicht wird, dass die Zelle die Flüssigkeit in ihrer Umgebung konditioniert. Die Behälterwand 41 kann durchsichtig sein, um eine allseitige Beobachtung der Zelle 1 in der Halterungseinrichtung 10 zu ermöglichen.

Die Lager 42 besitzen typische Durchmesser im Bereich von 50 µm bis zu einigen mm. Eine Abdichtung der Lager 42 ist bei geeigneter Dimensionierung, so dass Flüssigkeiten im Lager 42 durch Kapillarkräfte gehalten werden nicht erforderlich. Alternativ kann eine Dichtung, z. B. aus einem elastischen Material vorgesehen sein. Die Lager 42 können durch die Behälterwand 41 gebildet werden, indem diese mit einer ausreichenden Dicke, z. B. größer als 0,5 mm hergestellt wird. Damit wird der Aufbau des Behälters vereinfacht und die Bewegung der Trägerelemente stabilisiert.

In der Behälterwand 41 sind des Weiteren ein Fluidkanal 43, ein Sensor 44 und ein Optikelement 45 vorgesehen. Der Fluidkanal 43 umfasst zwei Leitungen, die an einander gegenüberliegenden Seiten der Behälterwand 41 in den Behälter 40 münden. Der Fluidkanal 43 ist vorgesehen, um eine Kultivierungsflüssigkeit durch den Behälter 40 zu strömen, eine zu untersuchende Zelle (z. B. 2) in den Haltebereich 11 zu spülen und/oder Zellen nach einem Test aus dem Behälter 40 zu entfernen. Der Fluidkanal 43 besitzt einen Durchmesser im Bereich von z. B. 50 µm bis zu einigen mm.

Der Sensor 44 (schematisch gezeigt) enthält z. B. Elektroden, die für eine Impedanzmessung an der Zelle 1 eingerichtet sind. Der Sensor 44 ist mit einem Messgerät (nicht dargestellt) verbunden. Das Optikelement 45 (schematisch gezeigt) umfasst z. B. ein optisches Fenster oder einen Anschluss für einen Lichtleiter für optische Untersuchungen (siehe auch Figur 2).

Die Konditionierungsvorrichtung 100 gemäß Figur 1 ist insbesondere zur biokompatiblen Halterung, Stimulation und/oder Aggregation von lebenden Zellen, insbesondere aus tierischen oder menschlichen Organismen, eingerichtet. In der Halteeinrichtung 10 wird die Zelle 1 so gehaltert, dass sie nicht mit biologisch wirksamen Substanzen, insbesondere Oberflächen in Berührung kommt. Zur Durchführung eines erfindungsgemäßen Verfahrens werden bspw. die folgenden Schritte realisiert. Zunächst wird eine zu untersuchende Zelle 1 über den Fluidkanal 43 (siehe Pfeil) in den Haltebereich 11 der Halteeinrichtung 10 eingeströmt und dort im Feldkäfig 12 gefangen. Wenn die Zelle 1 im Feldkäfig 12 positioniert ist, werden die Halteelemente 13 zu der Zelle 1 bewegt, so dass deren Halteflächen die Zelloberfläche berühren. Die Halteflächen bilden in diesem Fall einen Teil der Halteeinrichtung 10. Hierzu weisen die Stempelflächen eine biokompatible Beschichtung, z. B. aus Fibronektin oder Kollagen auf.

Nach Ausbildung eines molekularen Kontaktes zwischen der Zelloberfläche und den Halteflächen der Halteelemente 13, was nach einigen Minuten abgeschlossen sein kann, kann der Feldkäfig 12 abgeschaltet werden. In dieser Situation können die Elektrodenträger 12.3 zurückgezogen werden, so dass die Zelle 1 ausschließlich durch die Halteelemente 13 gehaltert wird. Vorteilhafterweise wird damit freier Raum für die Zuführung von Trägerelementen zum Haltebereich 11 geschaffen.

Anschließend werden Trägerelemente (z. B. 21, 22) zum Haltebereich 11 vorgeschoben. Auf den Stempelflächen 24 dieser Trägerelemente 21, 22 befinden sich Konditionierungsproben, die z. B. biologische Matrixmoleküle wie Laminin, Fibronektin oder Collagen, eine Schicht immobilisierter, funktionaler Makromoleküle, die auf einer natürlichen Zelloberfläche vorkommen, wie z. B. Integrine, Catherine, Signalrezeptoren und dgl., oder eine weitere Zelle 3 umfassen.

Die Berührung der Zelle 1 ist nicht auf die illustrierten Trägerelemente 21, 22 beschränkt, sondern entsprechend mit weiteren Trägerelementen möglich, die in weiteren Lagern in der Behälterwand 11 verschiebbar angeordnet sind.

Durch eine Steuerung der Antriebseinrichtung 50, insbesondere eine Zeitsteuerung der piezoelektrischen Antriebe (z. B. 51) kommt die Zelle 1 zeitlich und räumlich steuerbar mit vorbestimmten Konditionierungsproben in Kontakt. Dabei werden in Abhängigkeit von der Wirkung der Konditionierungsproben in der Zelle 1 Signalketten ausgelöst, die zu einer biochemischen Veränderung der Zelle führen. Die biochemische Veränderung der Zelle kann ggf. nach einer nachfolgenden Kultivierung außerhalb des Behälters 40 oder bereits unmittelbar in dem Haltebereich 11 durch geeignete Untersuchungen, z. B. optische oder elektrische Messungen charakterisiert werden.

Bei Bedarf können über den Fluidkanal 43 weitere Zellen (z. B. 2) zugeführt werden. Im Ergebnis können im Haltebereich 11 Zellen zu einem Zellaggregat zusammengefügt werden. Eine weitere Möglichkeit, Zellen zur Zelle 1 hinzuzufügen, besteht in der Verwendung eines der Trägerelemente (z. B. 22), auf dessen Stempelfläche ebenfalls eine Zelle 3 angeordnet sein kann.

Vorteilhafterweise stellt somit die Konditionierungsvorrichtung 100 ein universell und flexibel verwendbares System für eine zeitliche und räumliche Prägung von Zellen (z. B. Stammzellen) mit dem Ziel der Differenzierung und/oder der Gewebeinduktion dar.

Die illustrierten Ausführungsformen der Konditionierungsvorrichtung 100 und des Konditionierungsverfahrens können wie folgt modifiziert werden. In der Konditionierungsvorrichtung 100 können weitere Messeinrichtungen, wie z. B. eine pH-Messeinrichtung, ein Temperatursensor oder ein Lichtsensor angeordnet sein. Eine optische Messung kann insbesondere eine Streulichtmessung umfassen, mit der geometrische Eigenschaften der getesteten Zelle, wie z. B. die Größe oder die Oberflächenbeschaffenheit getestet werden können.

Die Konditionierungsvorrichtung 100 kann mit einer Regeleinrichtung ausgestattet sein, bei der die Antriebseinrichtung 50 in Abhängigkeit von Signalen der Messeinrichtung gesteuert wird.

Die Trägerelemente und ggf. Halteelemente können für eine Kraftmessung an der Zelle 1 verwendet werden. Damit können zwischenmolekulare Kräfte zwischen der Zellmembran und der Konditionierungsprobe erfasst werden. Des Weiteren können die Trägerelemente und ggf. die Halteelemente verwendet werden, um die zu testende Zelle in eine bestimmte Form zu bringen. Es können ferner Zellen zu Zellaggregaten zusammengesetzt werden.

Zellen oder Zellaggregate können anisotrop mit den Trägerelementen oder ggf. Halteelementen mit wechselnden Kräften beaufschlagt und mechanisch unter Stress gesetzt oder bewegt werden. Damit können physiologische Prozesse, wie z. B. eine Knochendeformation oder eine periodische Gewebedeformation nachgebildet werden.

Figur 2 zeigt in einer schematischen Schnittansicht eine abgewandelte Variante der Ausführungsform gemäß Figur 1, bei der als Optikelemente ein planes optisches Fenster 46 und Lichtleiter 47 vorgesehen sind. Durch das optische Fenster 46 kann die Zelle 1 mit einer externen Messeinrichtung 60, z. B. einer Kamera oder einem Mikroskop, von dem das Objektiv 61 illustriert ist, beobachtet werden. Über die Lichtleiter 47 erfolgt eine Beleuchtung der Zelle 1 von verschiedenen Seiten, um bspw. die Abbildung mit der Messeinrichtung 60 zu verbessern, eine Streulichtmessung zu ermöglichen oder Anregungslicht für eine Fluoreszenzmessung einzukoppeln.

Figur 2 illustriert des Weiteren, dass der Behälter 40 in einem Gefäß 70 angeordnet sein kann, das auch die Trägerelemente (z. B. 21, 22) und die piezoelektrischen Antriebe (nicht dargestellt) enthält und vollständig mit einer Kultivierungsflüssigkeit gefüllt ist. In diesem Fall ist es vorteilhafterweise nicht erforderlich, die Durchgangsöffnungen des Behälters 40, insbesondere die Lager 42 (siehe Figur 1) abzudichten.

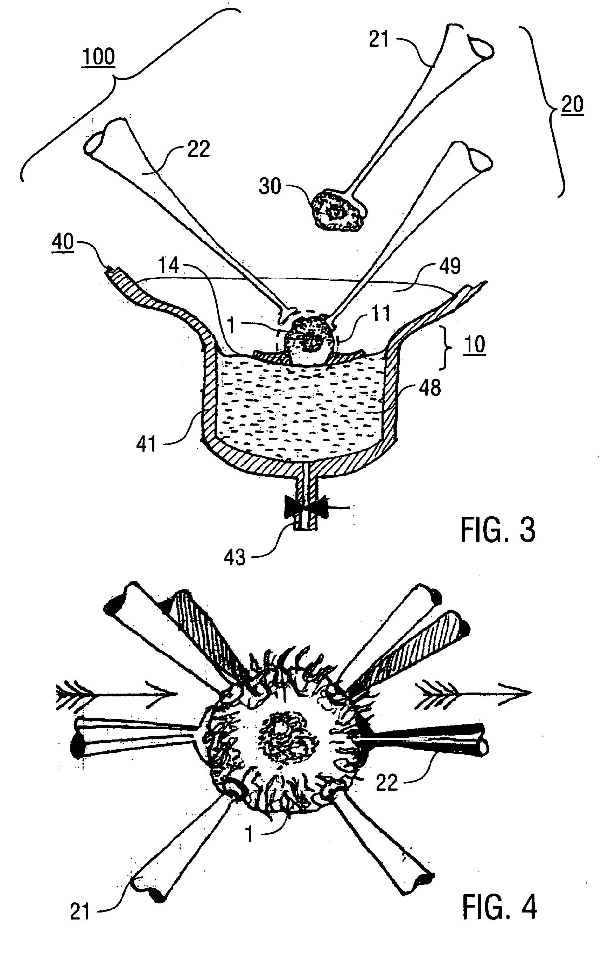

Figur 3 illustriert in schematischer Schnittansicht eine weitere Ausführungsform der erfindungsgemäßen Konditionierungsvorrichtung 100, bei der die Halteeinrichtung 10 eine Haltefläche 14 umfasst, auf der der Haltebereich 11 gebildet ist. Die Halteeinrichtung 10 ist in einem trichterförmigen Behälter 40 angeordnet. Die Haltefläche 14 ist zwischen Kultivierungsphasen gebildet, die eine untere Gelschicht 48 (z. B. aus Alginat) und eine obere Flüssigkeitsschicht 49 (z. B. aus einem Nährmedium) umfassen. Auf der Gelschicht 48 kann ein biokompatibles Matrixmaterial zur Eingrenzung des Haltebereichs 11 vorgesehen sein. Mit dem biokompatiblen Matrixmaterial, das z. B. PTFE oder ein anderes Polymer umfasst, kann die Lage der Zelle 1 stabilisiert oder vorgegeben werden. Im Haltebereich 11 können weitere Gele, biokompatible Polymere oder auch Testkörper mit biokompatibler Beschichtung angeordnet sein, um den Haltebereich 11 für die Zelle 1 zu definieren.

Die Haltefläche 14 kann alternativ durch die Grenzfläche zwischen zwei nicht-mischbaren Flüssigphasen gebildet werden, an der die Zelle 1 schwimmt. In diesem Fall wird die Fixierung der Zelle 1 im Haltebereich 11 zusätzlich durch Halteelemente stabilisiert.

Die untere Gelschicht 48 kann bei einer bevorzugten Anwendung der Erfindung, bei der zellbiologische Prozesse während der Embryogenese untersucht werden, eine biologische Phase wie z. B. das Eigelb mit umgebender Membran eines Vogeleis umfassen. Dabei kann die Form der Haltefläche 14 eben oder gekrümmt gebildet sein.

Bei der in Figur 3 gezeigten Ausführungsform der erfindungsgemäßen Konditionierungsvorrichtung 100 werden die Trägerelemente 21, 22 durch die obere Öffnung des Behälters 40 zu der Zelle 1 im Haltebereich 11 bewegt. Die Trägerelemente 21, 22 umfassen jeweils einen Trägerstab 25 und eine Stempelfläche 26, die sich am freien Ende des Trägerstabs 25 befindet und die Konditionierungsprobe 30, z. B. eine weitere biologische Zelle trägt.

Der Behälter 40 ist mit einem Fluidkanal 43 ausgestattet, der einen Zu- und/oder Abfluss zum Behälter 40 bildet und ein Ventil enthält. Durch den Fluidkanal 43 kann z. B. Nährlösung und/oder Trägerlösung zur Erzeugung der nicht-mischbaren Flüssigkeitsphasen eingeführt werden.

Figur 4 illustriert vergrößert eine Zelle 1, die von mehreren Trägerelementen 21, 22 allseitig berührt wird. Die Zelle 1 befindet sich in einer Kultivierungsflüssigkeit, die z. B. im Pfeilrichtung an der Zelle 1 vorbeiströmt. Die Kultivierungsflüssigkeit umfasst z. B. eine Nährlösung, eine Lösung mit mindestens einem Differenzierungsfaktor und/oder eine Lösung mit mindestens einem Signalfaktor.

Mindestens ein Trägerelement kann einen hohlen Trägerstab 25 umfassen, wie beispielhaft bei dem Trägerelement 22 gezeigt ist. Beispielsweise können hohe Nadeln als Trägerelemente verwendet werden, um die Konditionierungsprobe in flüssiger Form, z. B. als Lösung oder Suspension mit genetischen Materialien, Zellkernen oder deren Bestandteilen mit der zu testenden Zelle 1 in Kontakt zu bringen.

Figur 5 illustriert beispielhaft Varianten der erfindungsgemäß verwendeten Trägerelemente 21, die jeweils einen Trägerstab 25 und eine Stempelfläche 26 umfassen. Gemäß Figur 5A ist eine konvexe, kugelförmige Stempelfläche 26 vorgesehen, die als Konditionierungsprobe 30 z. B. Antikörper trägt. Die Antikörper sind auf der Stempelfläche 26 mit an sich bekannten Verfahren immobilisiert. Der Durchmesser der Stempelfläche 26 gemäß Figur 5A ist z. B. im Bereich von 50 nm bis 20 µm gewählt. Vorteilhafterweise wird dadurch die Kontaktfläche mit der zu testenden Zelle minimiert.

Gemäß Figur 5B ist eine ebene Stempelfläche 26 vorgesehen, die eine mit einer herkömmlichen in vitro-Kulturfläche vergleichbare Oberfläche darstellt. Der Durchmesser der Stempelfläche gemäß Figur 5B ist z. B. im Bereich von 50 µm bis 200 µm gewählt. Vorteile dieser Variante der Erfindung ergeben sich aus einem besonders großen Flächenkontakt mit der zu testenden Zelle und der Möglichkeit mit der Stempelfläche 26 die Zelle zu halten und in ihrer Position zu stabilisieren.

In Figur 5C ist beispielhaft eine konkave Stempelfläche 26 gezeigt, die von einem Randwulst 27 umgeben ist. Innerhalb der Randwulst 27 ist die Stempelfläche 26 mit einem Muster (z. B. Schachbrett-Muster) strukturiert. Die verschiedenen Bereiche im Muster umfassen z. B. räumlich getrennte, immobilisierte Makromoleküle, die z. B. vorbestimmte Signalfaktoren oder Differenzierungsfaktoren umfassen. Das Muster und die Auswahl der Konditionierungsproben kann in Abhängigkeit von der gewünschten Testaufgabe gewählt werden. Vorteilhafterweise wird mit der Variante gemäß Figur 5C der Zelle eine sog. molekulare Landschaft präsentiert, die ein Modell für ein molekulares Signalmuster einer anderen Zelle oder für eine definierte Differenzierung im Fall von Stammzellen darstellt.

Figur 6 illustriert eine weitere Variante des erfindungsgemäßen Konditionierungsverfahrens. In einer ersten Phase wird die Zelle 1 in Pfeilrichtung auf zwei Halteelemente 13 geströmt. Die Zelle 1 haftet auf den Halteflächen der Halteelemente 13 an. Anschließend können mit den Trägerelementen 21, 22 eine oder mehrere Konditionierungsproben 30 mit der Zelle 1 in Berührung gebracht werden. Diese Variante der Erfindung hat den Vorteil, dass auf die Bildung des dielektrischen Feldkäfigs (Figur 1) verzichtet werden kann. Der Aufbau der Konditionierungsvorrichtung 100 kann entsprechend vereinfacht werden.

Figur 7 zeigt schematisch eine getestete Zelle 1, auf deren Oberfläche ovale Oberflächenareale erkennbar sind, die mit Konditionierungsproben, z. B. molekularen Mustern in Berührung stehen oder standen. Die Berührung der Zelle 1 mit den verschiedenen Konditionierungsproben 30 kann gleichzeitig oder zeitlich versetzt zu verschiedenen Zeitpunkten erfolgt sein. Die Bereitstellung von Konditionierungsproben mit einer zeitlichen Abfolge kann für Untersuchungen von biologischen Prozessen, wie z. B. der Embryogenese oder der Wundheilung verwendet werden.

Figur 8 zeigt eine weitere Variante einer Zelle 1, die allseitig von einer Vielzahl von Trägerelementen (z. B. 21, 22) berührt wird. Wenn die lateralen Abstände zwischen den Stempelflächen benachbarter Trägerelemente größer oder gleich 1 µm ist, wird vorteilhafterweise vermieden, dass eine Zelle mit Zellkern den von den Stempelflächen der Trägerelemente gebildeten Hohlraum verlässt. Entsprechend können die Trägerelemente, ggf. in Zusammenwirkung mit Halteelementen (13, siehe Figuren 1, 6) die Positionierung der Zelle 1 in der Konditionierungsvorrichtung verbessern.

Vorteilhafterweise können für eine Zelluntersuchung einige bis zu einigen 100 Trägerelementen verwendet werden. Diese können bei geeigneter Wahl der Größe der Stempelflächen so kombiniert werden, dass die gesamte Zelle 1 von den Stempelflächen der Trägerelemente eingeschlossen ist. Die Trägerelemente und ggf. Halteelemente können zur Bildung eines dreidimensional definierten und in seiner Oberfläche vollständig bekannten Raumes verwendet werden.

Die in der vorstehenden Beschreibung, den Zeichnungen und den Ansprüchen offenbarten Merkmale der Erfindung können sowohl einzeln als auch in Kombination für die Verwirklichung der Erfindung in ihren verschiedenen Ausgestaltungen von Bedeutung sein.

## Patentansprüche

1. Konditionierungsvorrichtung (100) für biologische Zellen, umfassend:
- eine Halteeinrichtung (10), die zur Halterung von mindestens einer biologischen Zelle (1) in einem Haltebereich (11) eingerichtet ist, und
- eine Kontakteinrichtung (20), die zur Halterung von mindestens einer Konditionierungsprobe (30, 3) und zur Bewegung der mindestens einen Konditionierungsprobe (30, 3) zur Halteeinrichtung (10) derart eingerichtet ist, dass die mindestens eine Konditionierungsprobe (30, 3) die mindestens eine biologische Zelle (1) berührt,
**dadurch gekennzeichnet, dass**
- die Halteeinrichtung (10) zur biokompatiblen Halterung der biologischen Zelle (1) eingerichtet ist,
- die Kontakteinrichtung (20) mindestens zwei mechanische Trägerelemente (21, 22) aufweist, die aus verschiedenen Raumrichtungen zur Halteeinrichtung (10) beweglich sind.

2. Konditionierungsvorrichtung gemäß Anspruch 1, bei der
- die Halteeinrichtung (10) zur Halterung der mindestens einen biologischen Zelle (1) mit einer berührungslos wirkenden Kraft eingerichtet ist,
- die Halteeinrichtung (10) mindestens eine Haltefläche (14) aufweist, an der die mindestens eine biologische Zelle (1) positionierbar ist, und/oder
- die Halteeinrichtung (10) in einem Behälter (40) angeordnet ist, der zur Aufnahme eines flüssigen Kultivierungsmediums eingerichtet ist.

3. Konditionierungsvorrichtung gemäß Anspruch 2, bei der die Haltefläche (14)
- Teil mindestens eines mechanischen Halteelements (13) ist,
- eine Grenzfläche zwischen zwei Kultivierungsphasen (48, 49) umfasst, und/oder
- eine biokompatible Beschichtung aufweist, die für die mindestens eine biologische Zelle biochemisch unwirksam ist.

4. Konditionierungsvorrichtung gemäß Anspruch 2, bei der
- der Behälter (40) ein Innenvolumen aufweist, das größer als das vierfache Volumen der biologischen Zelle (1) ist, und/oder
- der Behälter (40) mindestens ein Lager (42), in dem mindestens eines der mechanischen Trägerelemente (21, 22) oder das mindestens eine mechanischen Halteelement (13) verschiebbar ist, mindestens einen Fluidkanal (43), durch den eine Fluidverbindung mit einer Umgebung des Behälters (40) hergestellt werden kann, mindestens einen Sensor (44), der zur Erfassung mindestens einer physikalischen und/oder chemischen Größe im Behälter (40) eingerichtet ist, und/oder mindestens ein Optikelement (45, 46, 47) umfasst, das zur Beleuchtung und/oder optischen Untersuchung im Behälter (40) eingerichtet ist.

5. Konditionierungsvorrichtung gemäß mindestens einem der vorhergehenden Ansprüche, bei der
- mindestens eines der Trägerelemente (21, 22) eine Stabform aufweist, und/oder
- mindestens eines der Trägerelemente (21, 22) eine Stempelfläche (26) aufweist, die zur Aufnahme der Konditionierungsprobe (30, 3) eingerichtet ist.

6. Konditionierungsvorrichtung gemäß Anspruch 5, bei der
- die Stempelfläche (26) kleiner als die Oberfläche der biologischen Zelle (1) ist, und/oder
- die Stempelfläche (26) eine konkave, konvexe oder ebene Form, eine Mikrostrukturierung und/oder eine Testbeschichtung aus biologisch wirksamen Molekülen aufweist.

7. Konditionierungsvorrichtung gemäß mindestens einem der vorhergehenden Ansprüche, die umfasst
- eine Antriebseinrichtung (50), mit der mindestens eines der Trägerelemente (21, 22) beweglich ist.

8. Konditionierungsvorrichtung gemäß Anspruch 7, bei der
- die Antriebseinrichtung (50) mindestens einen piezoelektrischen Antrieb (51) enthält, und/oder
- die Antriebseinrichtung (50) für eine radiale Bewegung des mindestens einen Trägerelements (21, 22) zu oder von dem Haltebereich (11) der Halterungseinrichtung (10) eingerichtet ist.

9. Konditionierungsvorrichtung gemäß mindestens einem der vorhergehenden Ansprüche, bei der
- mindestens zehn mechanische Trägerelemente (21, 22) vorgesehen sind.

10. Konditionierungsvorrichtung gemäß Anspruch 9, bei der
- die Trägerelemente (21, 22) für eine radial mehrseitige Berührung der mindestens einen biologischen Zelle (1) in der Halteeinrichtung (10) eingerichtet sind.

11. Konditionierungsverfahren zur Untersuchung einer Wechselwirkung von mindestens einer biologischen Zelle (1) mit mindestens einer Konditionierungsprobe (30, 3) unter Verwendung einer Konditionierungsvorrichtung (100) gemäß mindestens einem der vorhergehenden Ansprüche, umfassend die Schritte:
- Bereitstellung der mindestens einen biologischen Zelle (1) im Haltebereich (11) der Halteeinrichtung (10),
- Bereitstellung der mindestens einen Konditionierungsprobe (30, 3) auf mindestens einem der Trägerelemente (21, 22), und
- Bewegung von mindestens einem der Trägerelemente (21, 22) derart, dass die mindestens eine biologische Zelle (1) und die mindestens eine Konditionierungsprobe (30, 3) sich gegenseitig berühren.

12. Verfahren gemäß Anspruch 11, bei dem
- als weiterer Schritt eine Untersuchung der mindestens einen biologischen Zelle (1) vorgesehen ist,
- mehrere Konditionierungsproben gleichzeitig die mindestens eine biologische Zelle (1) berühren,
- mehrere Konditionierungsproben zeitlich versetzt die mindestens eine biologische Zelle (1) berühren,
- die Konditionierungsprobe (30, 3) aus der Gruppe ausgewählt ist, die biologisch wirksame Makromoleküle, Signalmoleküle, biologische Zellen, Zellbestandteile und mikrostrukturierte Oberflächen umfasst,
- die Bereitstellung der mindestens einen biologischen Zelle (1) mit einer optischen Pinzette, einem dielektrischen Käfig (12) oder einer Ultraschall-Falle erfolgt, und/oder
- das mindestens eine Trägerelement mit einer Geschwindigkeit bewegt wird, die im Bereich von 5 µm/h bis 10 mm/h gewählt ist.

13. Verfahren gemäß mindestens einem der Ansprüche 11 bis 12, bei dem
- im Haltebereich mehrere Zellen (1, 2) gruppiert werden.

14. Verfahren gemäß mindestens einem der Ansprüche 11 bis 13, mit mindestens einem der weiteren Schritte:
- Bereitstellung einer Konditionierungsprobe als Komponente einer Kultivierungsflüssigkeit, welche die mindestens eine biologische Zelle umgibt,
- Bildung eines die mindestens eine biologische Zelle umgebenden Hohlraums, der durch Stempelflächen (26) von Trägerelementen (21, 22) und/oder Halteflächen von Halteelementen (13) eingeschlossen wird,
- Messung von Wechselwirkungskräften zwischen der mindestens einen biologischen Zelle (1, 2) und der mindestens einen Konditionierungsprobe (30, 3),
- mechanische Deformation der mindestens einen biologischen Zelle (1),
- Flüssigkeitsbehandlung der mindestens einen biologischen Zelle (1),
- Kultivierung der mindestens einen biologischen Zelle (1),
- Verkapselung der mindestens einen biologischen Zelle (1), und
- Überführung der mindestens einen biologischen Zelle (1) in einen gefrorenen Zustand.

## Claims

1. Conditioning device (100) for biological cells, comprising:
- a holding device (10) adapted to hold at least one biological cell (1) in a holding area (11), and
- a contacting device (20) adapted to hold at least one conditioning sample (30, 3) and to move the at least one conditioning sample (30, 3) to the holding device (10) in such a manner that the at least one conditioning sample (30, 3) contacts the at least one biological cell (1),
**characterized in that**
- the holding device (10) is adapted for a biocompatible holding of the biological cell (1),
- the contacting device (20) has at least two mechanical carrier elements (21, 22) that can move from different spatial directions to the holding device (10).

2. Conditioning device according to claim 1, in which
- the holding device (10) is adapted to hold the at least one biological cell (1) with a force acting in a contactless manner,
- the holding device (10) has at least one holding surface (14) on which the at least one biological cell (1) can be positioned, and/or
- the holding device (10) is arranged in a container (40) adapted to receive a liquid cultivation medium.

3. Conditioning device according to claim 2, in which the holding surface (14)
- is part of at least one mechanical holding element (13),
- has a boundary surface between two cultivation phases (48, 49), and/or
- has a biocompatible coating that is biochemically inactive for the at least one biological cell.

4. Conditioning device according to claim 2, in which
- the container (40) has an inner volume that is greater than the fourfold volume of the biological cell (1), and/or
- the container (40) comprises at least one support (42) in which at least one of the mechanical carrier elements (21, 22) or the at least one mechanical holding element (13) can be shifted, at least one fluid conduit (43) through which a fluid connection can be established with an environment of the container (40), at least one sensor (44) that is adapted to detect at least one physical and/or chemical quantity in the container (40), and/or at least one optical element (45, 46, 47) that is adapted for an illumination and/or optical examination in the container (40).

5. Conditioning device according to at least one of the preceding claims, in which
- at least one of the carrier elements (21, 22) has a rod-shaped form, and/or
- at least one of the carrier elements (21, 22) has a stamping surface (26) that is adapted to receive the conditioning sample (30, 3).

6. Conditioning device according to claim 5, in which
- the stamping surface (26) is smaller than the surface of the biological cell (1), and/or
- the stamping surface (26) has a concave, convex or plane form, a microstructuring, and/or a test coating of biologically active molecules.

7. Conditioning device according to at least one of the preceding claims, that comprises
- a drive device (50), with which at least one of the carrier elements (21, 22) can be moved.

8. Conditioning device according to claim 7, in which
- the drive device (50) includes at least one piezoelectric drive (51), and/or
- the drive device (50) is adapted to radially move the at least one carrier element (21, 22) to or from the holding area (11) of the holding device (10).

9. Conditioning device according to at least one of the preceding claims, in which
- at least ten mechanical carrier elements (21, 22) are provided.

10. Conditioning device according to claim 9, in which
- the carrier elements (21, 22) are adapted for a radially multi-side contacting of the at least one biological cell (1) in the holding device (10).

11. A conditioning method for examining an interaction of at least one biological cell (1) with at least one conditioning sample (30, 3) using a conditioning device (100) in accordance with at least one of the preceding claims, comprising the steps:
- providing the at least one biological cell (1) in the holding area (11) of the holding device (10),
- providing the at least one conditioning sample (30, 3) on at least one of the carrier elements (21, 22), and
- moving at least one of the carrier elements (21, 22) in such a manner that the at least one biological cell (1) and the at least one conditioning sample (30, 3) mutually contact each other.

12. The method according to claim 11, in which
- examination of the at least one biological cell (1) is provided as a further step,
- several conditioning samples simultaneously contact the at least one biological cell (1),
- several conditioning samples contact the at least one biological cell (1) staggered in time,
- the conditioning sample (30, 3) is selected from the group comprising biologically active macromolecules, signal molecules, biological cells, cell components and microstructured surfaces,
- the providing of the at least one biological cell (1) takes place with an optical tweezer, a dielectric cage (12) or an ultrasonic trap, and/or
- the at least one carrier element is moved with a speed selected in the range of 5 µm/h to 10 mm/h.

13. The method according to at least one of claims 11 to 12, in which
- several cells (1, 2) are grouped in the holding area.

14. The method according to at least one of claims 11 to 13, with at least one of the further steps:
- providing a conditioning sample as component of a cultivation liquid that surrounds the at least one biological cell,
- forming of a hollow space surrounding the at least one biological cell, which hollow space is enclosed by stamping surfaces (26) of carrier elements (21, 22) and/or holding surfaces of holding elements (13),
- measuring of interactive forces between the at least one biological cell (1, 2) and the at least one conditioning sample (30, 3),
- mechanically deforming the at least one biological cell (1),
- liquid treatment of the at least one biological cell (1),
- cultivating of the at least one biological cell (1),
- encapsulating of the at least one biological cell (1), and
- transferring of the at least one biological cell (1) into a frozen state.

## Revendications

1. Dispositif de conditionnement (100) de cellules biologiques, comprenant :
- un dispositif de retenue (10) qui est conçu pour retenir au moins une cellule biologique (1) dans une zone de fixation (11), et
- un dispositif de mise en contact (20) qui est conçu pour le maintien d'au moins un échantillon de conditionnement (30, 3) et pour le déplacement d'au moins un échantillon de conditionnement (30, 3) pour le dispositif de retenue (10) de telle sorte qu'au moins un échantillon de conditionnement (30, 3) touche au moins une cellule biologique (1),
**caractérisé en ce que**
- le dispositif de retenue (10) est conçu pour le maintien biocompatible de la cellule biologique (1),
- le dispositif de mise en contact (20) présente au moins deux éléments porteurs mécaniques (21, 22) qui sont mobiles dans diverses orientations spatiales vers le dispositif de retenue (10).

2. Dispositif de conditionnement selon la revendication 1, dans lequel
- le dispositif de retenue (10) est conçu pour retenir au moins une cellule biologique (1) avec une force agissant sans contact,
- le dispositif de retenue (10) présente au moins une surface de retenue (14) sur laquelle au moins la cellule biologique (1) peut être placée, et/ou
- le dispositif de retenue (10) est disposé dans un récipient (40) qui est conçu pour recevoir un milieu de culture liquide.

3. Dispositif de conditionnement selon la revendication 2, dans lequel la surface de retenue (14)
- fait partie d'au moins un élément de retenue mécanique (13),
- comprend une interface entre deux phases de culture (48, 49), et/ou
- comprend un revêtement biocompatible qui est biochimiquement inactif pour au moins la cellule biologique.

4. Dispositif de conditionnement selon la revendication 2, dans lequel
- le récipient (40) présente un volume interne qui est supérieur à quatre fois le volume de la cellule biologique (1),
et/ou
- le récipient (40) est au moins un stockage (42), dans lequel au moins l'un des éléments porteurs mécaniques (21, 22) ou au moins l'élément de retenue mécanique (13) peut être décalé, au moins un canal de fluide (43) par lequel une liaison fluide peut être produite avec un environnement du récipient (40), au moins un capteur (44) qui est conçu pour saisir au moins une grandeur physique et/ou chimique dans le récipient (40) et/ou au moins un élément optique (45, 46, 47) qui est conçu pour l'éclairage et/ou pour l'examen optique dans le récipient (40).

5. Dispositif de conditionnement selon au moins l'une des revendications précédentes, dans lequel
- au moins l'un des éléments de support (21, 22) présente une forme de bâtonnet, et/ou
- au moins l'un des éléments de support (21, 22) présente une surface de poinçon (26) qui est conçue pour recevoir l'échantillon de conditionnement (30, 3).

6. Dispositif de conditionnement selon la revendication 5, dans lequel
- la surface de poinçon (26) est inférieure à la surface de la cellule biologique (1), et/ou
- la surface de poinçon (26) présente une forme concave, convexe ou plane, une microstructure et/ou un revêtement de test de molécules biologiquement actives.

7. Dispositif de conditionnement selon au moins l'une des revendications précédentes, qui comprend
- un dispositif d'entraînement (50) avec lequel au moins l'un des éléments de support (21, 22) peut être déplacé.

8. Dispositif de conditionnement selon la revendication 7, dans lequel
- le dispositif d'entraînement (50) contient au moins un entraînement piézoélectrique (51), et/ou
- le dispositif d'entraînement (50) est conçu pour un mouvement radial d'au moins un élément de support (21, 22) vers ou à partir de la zone de retenue (11) du dispositif de retenue (10).

9. Dispositif de conditionnement selon au moins l'une des revendications précédentes, dans lequel
- au moins dix éléments de support mécaniques (21, 22) sont prévus.

10. Dispositif de conditionnement selon la revendication 9, dans lequel
- les éléments de support (21, 22) sont conçus pour un contact radial multiple d'au moins une cellule biologique (1) dans le dispositif de retenue (10).

11. Procédé de conditionnement pour l'analyse d'une interaction d'au moins une cellule biologique (1) avec au moins un échantillon de conditionnement (30, 3) en utilisant un dispositif de conditionnement (100) selon au moins l'une des revendications précédentes, comprenant les étapes de :
- mise à disposition d'au moins une cellule biologique (1) dans la zone de retenue (11) du dispositif de retenue (10),
- mise à disposition d'au moins un échantillon de conditionnement (30, 3) sur au moins l'un des éléments de support (21, 22), et
- mouvement d'au moins l'un des éléments de support (21, 22) de telle sorte qu'au moins une cellule biologique (1) et au moins l'échantillon de conditionnement (30, 3) soient réciproquement en contact.

12. Procédé selon la revendication 11, dans lequel
- une analyse d'au moins une cellule biologique (1) est prévue comme autre étape,
- plusieurs échantillons de conditionnement sont en contact de façon concomitante avec au moins la cellule biologique (1),
- plusieurs échantillons de conditionnement sont en contact de façon décalée dans le temps avec au moins la cellule biologique (1),
- l'échantillon de conditionnement (30, 3) est choisi dans le groupe comprenant la macromolécule biologiquement active, une molécule de signal, des cellules biologiques, des composants cellulaires et des surfaces microstructurées,
- la mise à disposition d'au moins une cellule biologique (1) s'effectue avec une pincette optique, une cage diélectrique (12) ou un piège à ultrasons, et/ou
- au moins l'élément de support est déplacé à une vitesse qui est choisie dans une plage de 5 µm/h à 10 mm/h.

13. Procédé selon au moins l'une des revendications 11 à 12, dans lequel
- dans la zone de retenue, plusieurs cellules (1, 2) sont groupées.

14. Procédé selon au moins l'une des revendications 11 à 13, comportant au moins l'une des autres étapes :
- mise à disposition d'un échantillon de conditionnement comme composant d'un liquide de culture qui entoure au moins la cellule biologique,
- formation d'un espace creux entourant au moins la cellule biologique qui est circonscrit par des surfaces de pinçon (26) des éléments de support (21, 22) et/ou des surfaces de retenue des éléments de support (13),
- mesure des forces d'interaction entre au moins la cellule biologique (1, 2) et au moins un échantillon de conditionnement (30, 3),
- déformation mécanique d'au moins la cellule biologique (1),
- traitement liquide d'au moins la cellule biologique (1),
- culture d'au moins la cellule biologique (1),
- encapsulation d'au moins la cellule biologique (1), et
- passage d'au moins la cellule biologique (1) à un état congelé.
